# EUROPEAN PATENT SPECIFICATION

(11) **EP 2 749 229 B1**
(45) Date of publication and mention of the grant of the patent: **28.06.2017**
(21) Application number: 13194203.9
(22) Date of filing: 25.11.2013
(51) Int. Cl.: A61B 8/02, A61B 8/08, A61B 8/00

(54) **Ultrasound system and method of controlling the same**
Ultraschallsystem und Steuerungsverfahren dafür
Système à ultrasons et son procédé de contrôle

(30) Priority: 28.12.2012 KR 20120156147
(43) Date of publication of application: 02.07.2014
(73) Proprietor: Samsung Medison Co., Ltd., Gangwon-do 250-870 (KR)
(72) Inventor: Yoo, Jun Sang, Suwon-Si, Gyeonggi-Do (KR)
(74) Representative: Schmid, Wolfgang

(56) References cited:
- WO-A1-2005/110236
- US-A- 4 413 629
- US-A1- 2005 124 878
- US-A1- 2010 274 145

## Description

### TECHNICAL FIELD

The present disclosure relates to an ultrasound system to convert an ultrasound signal acquired from a target object into a sensory signal to output the sensory signal, and a method of controlling the ultrasound system.

### BACKGROUND

An ultrasound diagnosis apparatus has been extensively used in the medical field to acquire internal information of a target object due to its non-invasive and nondestructive nature. In particular, the ultrasound diagnosis apparatus may image a state of a fetus to show the image to a pregnant woman or may allow the pregnant woman to listen to the heartbeat of the fetus as a sound using a Doppler signal. A state of the fetus may be diagnosed. Further, the pregnant woman may have a moving experience and develop a bond with the fetus.

Such a service for providing a sensory output of the state of the fetus is valuable to pregnant women, and thus, there is a need to develop various technologies for providing a haptic output of the state of the fetus as well as an auditory or visual output of the state of the fetus.

The WO 2005/110236 A1 describes a beltless device for monitoring labor contractions via a fiber optic cable and fetal heartbeats via an ultrasound sensor. The same or separate fiber optic cables may be used to provide a visual Indication of labor contractions and fetal heartbeats. The device comfortably attaches to the abdomen of a pregnant mother and is preferably wireless to allow full mobility to the mother. The device may also be used to monitor heartbeats of children and adults.

In the US 2010/274145 A1 fetal and/or maternal monitoring devices, systems and methods using UWB medical radar are described. These devices and systems may include a UWB sensor providing high-resolution and reliable simultaneous monitoring of multiple indicators of fetal and/or maternal health, such as fetal heart rate, fetal heart rate variability, fetal respiration, fetal gross body movement, maternal contractions, maternal heart rate, maternal respiration, and other derivative parameters during virtually all stages of pregnancy and during delivery. The sensor allows novel collection of physiological data using a single sensor or multiple sensors to develop individual and aggregate normal motion indices for use in determining when departure from normal motion index is indicative of fetal or maternal distress.

The US 2005/124878 A1 discloses a fetal diagnostic apparatus which comprises ultrasonic imaging apparatus for producing ultrasonic images, the images comprise a multiplicity of pixels; an ultrasonic transducer that can be placed upon a patient, in data communication with the ultrasonic imaging apparatus; and a processor in data communication with the ultrasonic imaging apparatus that measures changes in the pixels with respect to time.

The US 4 413 629 A describes a portable ultrasonic Doppler System for sensing movement, e.g. fetal heartrate, within a body. A handheld unit includes a housing containing an ultrasonic transmitter and an ultrasonic receiver. The ultrasonic receiver detects ultrasonic energy emitted from the ultrasonic transmitter and reflected off of the moving object being monitored. The handheld unit also includes a radio frequency transmitter for transmitting a carrier modulated in accordance with the response of the ultrasonic receiver. A radio frequency receiver unit, separate from the handheld unit, receives the signal broadcast by the handheld unit. The receiver unit drives a speaker or earphone to provide an audio output for listening to the moving object being monitored. Digital display and memory circuitry is also provided which can be used in conjunction with said receiver unit to store and display data indicative of the movement being monitored. In conjunction with the handheld unit, a patient being monitored becomes the radio frequency antenna and the doctor becomes an extension of the antenna ground plane, providing a highly efficient radio link between the handheld unit and the receiver unit.

### SUMMARY

The invention is defined by claim 1. Regarding the method the invention is defined by claim 11.

An aspect of the present disclosure provides an ultrasound system and a method of controlling the same, which convert a bio-signal or a movement signal of a target object into a sensory signal including visual, auditory, and haptic signals to output the sensory signal, and thus, a state of the target object may experience through various senses.

Additional aspects of the disclosure will be set forth in part in the description which follows and, in part, will be obvious from the description, or may be learned by practice of the disclosure.

In accordance with an exemplary embodiment of the present disclosure, an ultrasound system includes a signal acquisitioner to acquire an ultrasound signal of a target object. A signal generator generates a bio-signal or a movement signal of the target objet from the ultrasound signal. A signal converter converts the bio-signal or the movement signal of the target object into a sensory signal, and a signal transmitter transmits the sensory signal to a haptic device.

The sensory signal may include a haptic signal.

The sensory signal may further include an auditory signal.

The signal transmitter may transmit, as a visual signal, the ultrasound signal and the bio-signal or the movement signal to the haptic device.

The bio-signal may be a heartbeat signal, and the signal converter may convert the bio-signal of the target object into a digital signal having signal characteristics of the bio-signal and may convert a pulse signal into the sensory signal.

The bio-signal may be a heartbeat signal, and the signal converter may convert the bio-signal or the movement signal of the target object into the sensory signal with different intensities according to an intensity of the bio-signal.

The bio-signal may be a heartbeat signal, and the signal converter may convert the bio-signal or movement signal of the target object into the sensory signal with different output times according to an intensity of the bio-signal.

The signal generator may generate the movement signal of the target object using a tracking algorithm.

The ultrasound system may further include an input to set a movement recognition target and a reference line.

The signal generator may generate the movement signal based on a relationship between the movement recognition target and the reference line.

The signal generator may generates the movement signal when the movement recognition target and the reference line approach each other such that a distance there between is equal to or less than a preset distance.

The ultrasound system may further include the haptic device to receive the sensory signal transmitted from the signal transmitter and to haptically and audibly output the sensory signal.

The ultrasound system may further include an input to set a response sensitivity of the haptic device with respect to the bio-signal or movement signal of the target object.

The ultrasound system may further include a signal storage to store the sensory signal, wherein the sensory signal stored in the signal storage may be loaded and transmitted to the haptic device through the signal transmitter.

The haptic device may include a signal receiver to receive the sensory signal from the signal transmitter, a signal storage to store the sensory signal, and an actuator driven according to the sensory signal when the sensory signal stored in the signal storage is loaded.

In accordance with another aspect of the present disclosure, a method of controlling an ultrasound system includes acquiring an ultrasound signal of a target object and generating a bio-signal or movement signal of the target objet from the ultrasound signal. The bio-signal or movement signal of the target object is converted into a sensory signal, and the sensory signal is transmitted to a haptic device.

The converting of the bio-signal of the target object into the sensory signal may include converting the bio-signal of the target object into a pulse signal, and converting the pulse signal into the sensory signal.

The bio-signal may be a heartbeat signal, and the converting of the bio-signal of the target object into the sensory signal may include converting the bio-signal of the target object into the sensory signal with different intensities according to an intensity of the bio-signal.

The bio-signal may be a heartbeat signal, and the converting of the bio-signal of the target object into the sensory signal may include converting the bio-signal of the target object into the sensory signal with different output times according to an intensity of the bio-signal.

The generating of the movement signal of the target object may include generating the movement signal of the target object using a tracking algorithm.

The method may further include setting a movement recognition target and a reference line in order to generate the movement signal of the target object.

The generating of the movement signal of the target object may include generating the movement signal based on a relationship between the movement recognition target and the reference line.

The generating of the movement signal of the target object may include generating the movement signal when the movement recognition target and the reference line approach each other such that a distance there between is equal to or less than a preset distance.

The method may further include receiving the sensory signal through the haptic device and haptically and audibly outputting the sensory signal.

The method may further include setting a response sensitivity of the haptic device with respect to the bio-signal or movement signal of the target object.

The method may further include storing the sensory signal, wherein the transmitting of the sensory signal to the haptic device may include loading the sensory signal and transmitting the sensory signal to the haptic device.

The receiving the sensory signal through the haptic device and the haptically and audibly outputting the sensory signal may include storing the sensory signal, and driving an actuator according to the sensory signal when the sensory signal is loaded.

### BRIEF DESCRIPTION OF THE DRAWINGS

These and/or other aspects of the disclosure will become apparent and more readily appreciated from the following description of the embodiments, taken in conjunction with the accompanying drawings.
FIG. 1 is a control block diagram of an ultrasound system according to an embodiment of the present disclosure.
FIG. 2 is a diagram showing an external appearance of an overall structure of an ultrasound system according to an embodiment of the present disclosure.
FIG. 3 is a control block diagram showing a structure of a signal acquisitioner.
FIG. 4 is a diagram showing a fetal heartbeat signal generated by a signal generator.
FIG. 5A is a diagram showing a pulse signal based on a fetal heartbeat signal.
FIG. 5B is a diagram showing a haptic signal converted from a pulse signal.
FIG. 6A is a graph showing a signal with vibration, intensity of which varies according to an intensity of a heartbeat signal.
FIG. 6B is a graph showing a signal with vibration, a period of time of which varies according to an intensity of a heartbeat signal.
FIG. 7 is a control block diagram of an ultrasound system that further includes an input and a display.
FIGS. 8A and 8B are diagrams related to generation of a fetal movement signal.
FIG. 9 is a control block diagram of the ultrasound system to store and load a bio-signal or movement signal of a target object.
FIG. 10 is a flowchart of a method of controlling an ultrasound system, according to an embodiment of the present disclosure.
FIG. 11 is a flowchart of a method of controlling an ultrasound system to express fetal movement using a tracking algorithm and a collision detection algorithm.

### DETAILED DESCRIPTION

Reference will now be made in detail to embodiments of the present disclosure, examples of which are illustrated in the accompanying drawings.

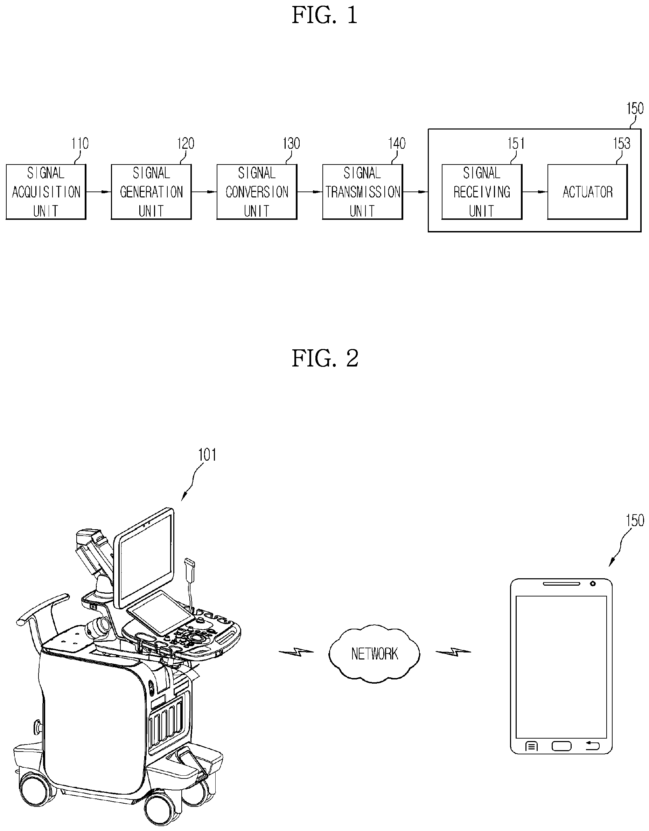

FIG. 1 is a control block diagram of an ultrasound system 100 according to an embodiment of the present disclosure and FIG. 2 is a diagram showing an external appearance of an overall structure of the ultrasound system 100 according to an embodiment of the present disclosure.

Referring to FIG. 1, the ultrasound system 100 includes a signal acquisitioner 110 to acquire an ultrasound signal of a target object, and a signal generator 120 to generate a bio-signal or movement signal of the target objet from the ultrasound signal. A signal converter 130 converts the bio-signal or movement signal of the target object into a sensory signal, and a signal transmitter 140 transmits the sensory signal to a haptic device 150 A haptic device 150 implements the transmitted sensory signal in a sensory form. Here, the sensory signal may include a haptic signal and may further include an auditory signal or visual signal as necessary.

The signal acquisitioner 110, the signal generator 120, the signal converter 130, and the signal transmitter 140 may be included in an ultrasound diagnosis apparatus 101. Thus, referring to FIG. 2, the haptic device 150 is connected to the ultrasound diagnosis apparatus 101 through a wireless or wired network to receive the haptic signal.

The haptic device 150 includes a signal receiver 151 to receive the haptic signal and an actuator 153 driven according to the received haptic signal. The haptic device 150 may be a device realized by adding a haptic function to a portable device such as a portable phone or a separate device to provide haptic feedback. Thus, the haptic device 150 may be any device to provide haptic feedback by driving the transmitted haptic signal, and thus, a type thereof is not particularly limited.

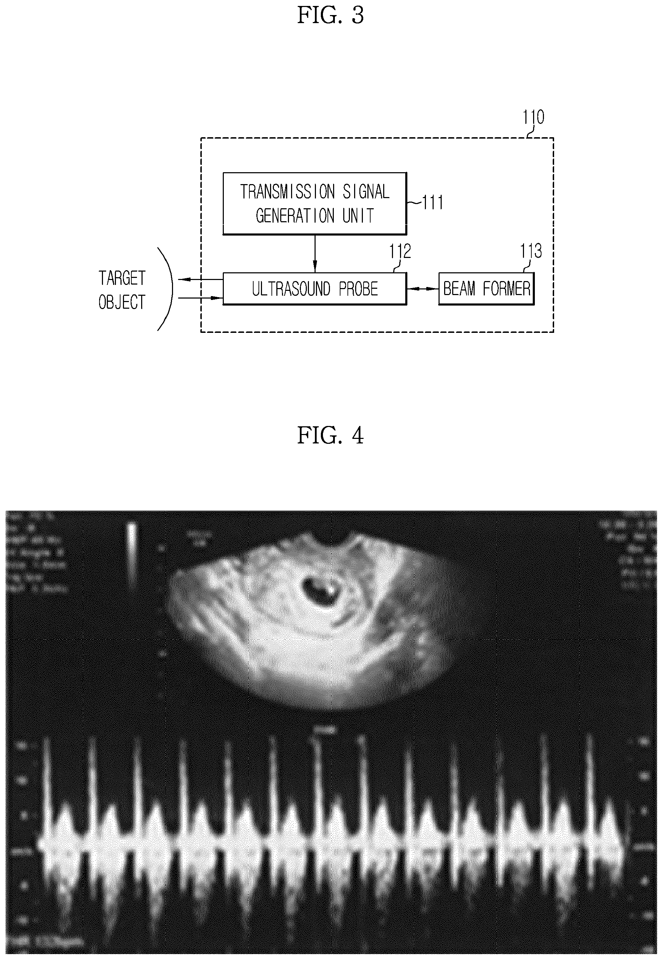

FIG. 3 is a control block diagram showing a structure of the signal acquisition unit 110. Referring to FIG. 3, the signal acquisitioner 110 includes a transmission signal generator 111 to generate a transmission signal to be transmitted to the target object. An ultrasound probe 112 receives the transmission signal from the transmission signal generator 111, converts the transmission signal into an ultrasound signal, and transmits and receives the ultrasound signal to and from the target object. A beam former 113 receives an ultrasound echo signal from the ultrasound probe 112 to generate a receiving focus signal.

The ultrasound probe 112 includes a plurality of transducers to interconvert the ultrasound signal and an electrical signal, and a driver to drive a transducer to swing. The driver may be a stepping motor for control of a rotation angle. When the transmission signal is transmitted from the transmission signal generator 111, the transducer converts the transmitted transmission signal into an ultrasound signal to transmit the ultrasound signal to the target object and receives an ultrasound echo signal reflected by the target object to generate a receiving signal. In this case, the receiving signal is an analog signal.

In detail, the ultrasound probe 112 appropriately delays an input time of pulses to each transducer to transmit focused ultrasound beams to the target object along a transmission scan line. Ultrasound echo signals reflected by the target object are input to each transducer at different receiving times and each transducer outputs the input ultrasound echo signals.

When the ultrasound probe 112 transmits ultrasound waves, the beam former 113 adjusts driving timing of each transducer of the ultrasound probe 112 to focus the ultrasound waves at a specific location. When the receiving signal is transmitted from the ultrasound probe 112, the beam former 113 converts the analog receiving signal into a digital signal. In addition, the beam former 113 focuses the digital signal to generate the receiving focus signal in consideration of a position and focusing point of the transducer. Hereinafter, the ultrasound signal according to the above-described embodiment may be the receiving focus signal output from the beam former 113 or an ultrasound image signal on which an imaging process is performed in order to image the target object.

The signal generator 120 generates the bio-signal or movement signal of the target object from the ultrasound signal of the target object. Generation of the bio-signal of the target object will be described with regard to an embodiment of the present disclosure.

FIG. 4 is a diagram showing a fetal heartbeat signal generated by the signal generator 120.

There are various bio-signals of the target object. For example, when the target object is a fetus, the bio-signal may be a heartbeat signal. In this case, Doppler Effect may be used in order to generate the fetal heartbeat signal from an ultrasound signal. Ultrasound waves irradiated from the ultrasound probe 112 into a human body collide with a moving object inside the human body and are reflected back to the ultrasound probe 112. The irradiated ultrasound waves and the reflected ultrasound waves have different frequencies. Such frequency change is called Doppler shift, and a phenomenon in which the Doppler shift occurs refers to the Doppler Effect.

In detail, ultrasound waves irradiated from the ultrasound probe 112 into the abdomen of a pregnant woman are reflected by a tissue, which moves in synchronization with fetal heartbeat of a heart wall, a blood vessel wall, blood, or the like, back to the ultrasound probe 112, and the frequency of the reflected ultrasound waves is shifted. Thus, the signal generator 120 may extract a signal, the frequency of which is shifted, from the ultrasound signal to generate the fetal heartbeat signal shown in FIG. 4.

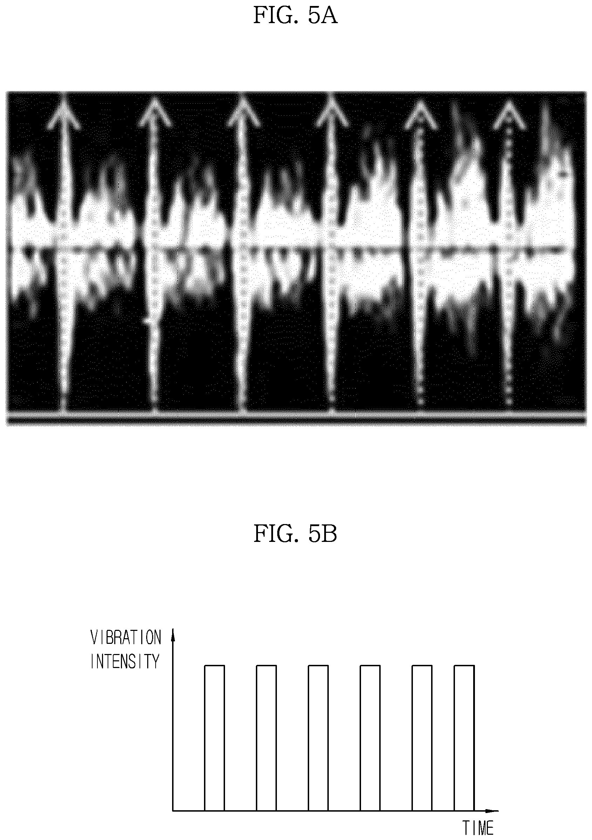

FIG. 5A is a diagram showing a pulse signal based on the fetal heartbeat signal and FIG. 5B is a diagram showing a haptic signal converted from the pulse signal.

The signal converter 130 converts the heartbeat signal of FIG. 4 into the haptic signal. Referring back to FIG. 4, the fetal heartbeat signal has periodicity according to ventricular systole. Thus, the signal converter 130 may generate a simple digital signal shown in FIG. 5A, for example, a pulse signal based on ventricular systole indicated in the heartbeat signal. The digital signal contains the characteristics of the heartbeat signal.

In addition, the signal converter 130 generates the haptic signal shown in FIG. 5B based on the generated pulse signal. The haptic signal is a signal to drive the haptic device 150 to vibrate or provide haptic feedback. The signal transmitter 140 transmits the haptic signal to the haptic device 150 through a wired or wireless network. In addition, the haptic signal is used to provide haptic feedback through the haptic device 150.

The haptic sense may include all senses obtained from external mechanical, thermal, chemical, and electric stimuli applied to skin, muscle, tendon, and joint of a human body and may be classified into tactile sense and kinesthetic sense.

The tactile sense is the sense of touch where the geometric shape, roughness, temperature, and slip of a contact surface are recognized via sense of the skin spread in or around the knuckle, palm, or the like. The kinesthetic sense is the sense of touch where total contact force, flexibility, weight, or the like is recognized via proprioception of bone and joint as well as muscle of the fingers, wrists, arms, or the like.

As an example, when the haptic device 150 vibrates according to the haptic signal shown in FIG. 5B, a user may feel the fetal heartbeat through the vibration.

The haptic device 150 may provide other feedback such as visual and auditory feedback together with the haptic feedback. To this end, the signal converter 130 may convert the heartbeat signal that is a bio-signal into an auditory signal and may transmit the converted auditory signal to the haptic device 150 through the signal transmitter 140. In addition, the haptic device 150 may output fetal heartbeat sound through a speaker.

In addition, the ultrasound signal, that is, a fetus image signal acquired by the signal acquisitioner 110 may also be transmitted to the haptic device 150 through the signal transmitter 140. The haptic device 150 may visually output the transmitted fetus image signal. In addition, a Doppler signal generated by the signal generator 120 may also be transmitted to the haptic device 150 through the signal transmitter 140 and may be output in the form of graph through a display. In this regard, when the Doppler signal is output together with the fetus image signal, the image shown in FIG. 4A may be output through the haptic device 150. However, embodiments of the present disclosure are not limited thereto, and any visual signal related to a state of the fetus may be output through the haptic device 150.

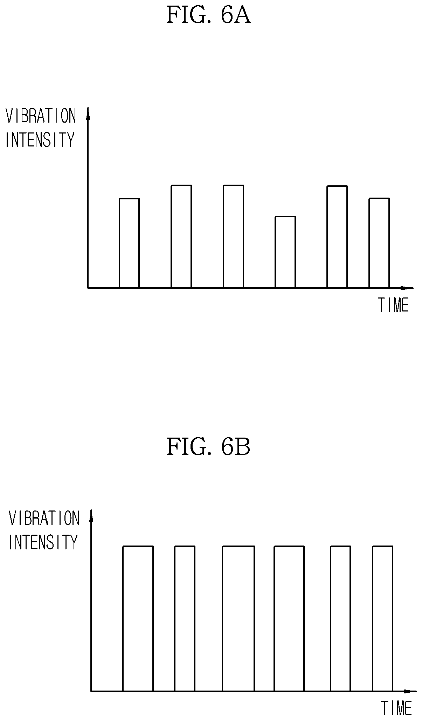

FIG. 6A is a graph showing a signal with vibration, the intensity of which varies according to an intensity of a heartbeat signal. FIG. 6B is a graph showing a signal with vibration, a period of time of which varies according to an intensity of a heartbeat signal.

The haptic signal of FIG. 5B indicates only a period of a fetal heartbeat signal. In an ultrasound system according to an embodiment of the present disclosure, the intensity of the fetal heartbeat as well as a period of the fetal heartbeat may be haptically expressed. As an example, the intensity of the vibration signal may vary according to the intensity of the fetal heartbeat signal, as shown in FIG. 6A. Thus, the haptic device 150 outputs the vibration signal, the intensity of which is increased as the intensity of the fetal heartbeat is increased.

As another example, a period of time when vibration occurs may vary according to the intensity of the fetal heartbeat signal, as shown in FIG. 6B. Thus, the haptic device 150 outputs the vibration signal, a period of time of which is increased as the intensity of the fetal heartbeat is increased.

The haptic device 150 may express the fetal heartbeat through kinesthetic sense as well as haptic sense such as the aforementioned vibration. As an example, the haptic device 150 may adopt a method in which liquid is concentrated on a specific location of the haptic device 150 to expand. In this regard, the fetal heartbeat may be expressed by repeating expansion and contraction according to the haptic signal shown in FIG. 5B. The location where liquid is concentrated may be a location within a user's reach, a heart location of a fetus image displayed on a display of the haptic device 150, or an arbitrary location determined by the user.

In addition, it may be possible to express only a period of the fetal heartbeat according to the signal shown in FIG. 5B, to express liquid with different expansion degrees according to the intensity of the fetal heartbeat using the signal shown in FIG. 6A, or to express liquid with different expansion times according to the intensity of the fetal heartbeat using the signal shown in FIG. 6B.

As described above, an auditory signal may also be output through the haptic device 150. In this regard, the fetal heartbeat may be audibly expressed, that is, may be expressed as a sound together with haptic sense. In this case, the sound with different intensities or different output times may be expressed according to the intensity of the fetal heartbeat.

The embodiments described with reference to FIGS. 4 through 6 relate to an ultrasound system to express fetal heartbeat through senses such as haptic sense or the like when a target object is a fetus. Hereinafter, an ultrasound system to express movement of a fetus (i.e., fetal movement) through senses such as haptic sense or the like will be described.

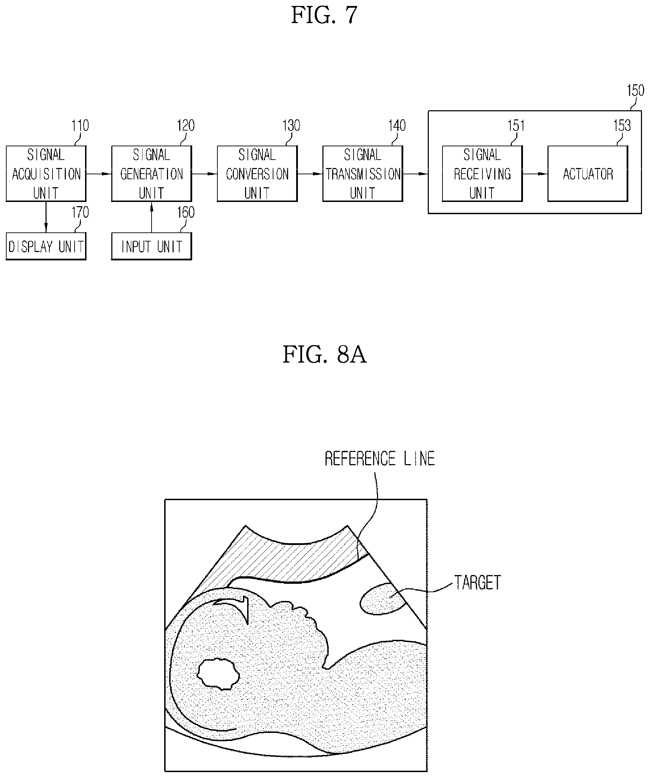

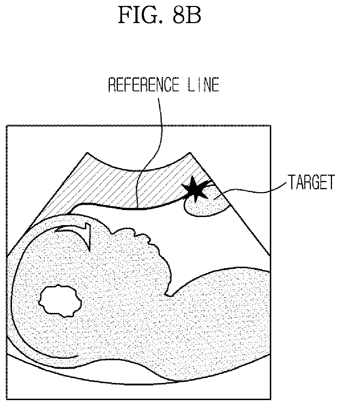

FIG. 7 is a control block diagram of an ultrasound system that further includes an input 160 and a display 170, and FIGS. 8A and 8B are diagrams related to generation of a fetal movement signal.

Referring to FIG. 7, the ultrasound system 100 described with reference to FIG. 1 may further include the input 160 to receive a user's command related to signal generation, and the display 170 displays an internal image of a target object. The signal generator 120 may generate the fetal movement signal. To this end, for example, a tracking algorithm or a collision detection algorithm may be used.

The ultrasound signal acquired by the signal acquisitioner 110 may include a two-dimensional or three-dimensional ultrasound signal of the fetus. As shown in FIG. 8A, when the fetus ultrasound signal acquired by the signal acquisitioner 110 is output through the display 170, the user sets a reference line and a movement recognition target through the input 160. The input 160 may be implemented as a keyboard, a trackball, a mouse, a touch panel, or the like. When the display 170 is implanted as a touchscreen, the touchscreen may perform functions of both the display 170 and the input 160.

The input 160 and the display 170 may be included in the ultrasound diagnosis apparatus 101 or the haptic device 150, or may be included in each of the ultrasound diagnosis apparatus 101 and the haptic device 150.

The signal generator 120 may recognize movement of the movement recognition target in real time using a tracking algorithm such as particle tracking, Kalman tracking, Blob detection, or the like.

In addition, the signal generator 120 determines whether a relationship between the reference line and the movement recognition target satisfies a preset condition. Here, the preset condition is a condition to determine whether the movement recognition target moves based on the reference line. For example, a case in which the reference line and the movement recognition target approach each other such that a distance there between may be equal to or less than a preset distance, or a case in which the movement recognition target reaches the reference line may be set as a condition for generation of a haptic signal.

For example, in the latter case, when movement of the movement recognition target is tracked in real time, if the movement recognition target reaches, that is, collides with the reference line, as shown in FIG. 8B, the signal generator 120 generates a movement signal indicating the fetal movement and the signal converter 130 converts the movement signal into the haptic signal such as a vibration signal and transmits the haptic signal to the haptic device 150 through the signal transmitter 140.

The signal converter 130 may convert the fetal movement signal into an auditory signal and transmit the auditory signal to the haptic device 150 through the signal transmitter 140. In this case, the auditory signal may be a signal indicating collision of the movement recognition target with the reference line or other signals from which the fetal movement may be detected.

In addition, a fetus ultrasound image or a fetal movement image shown in FIGS. 8A and 8B may also be transmitted to the haptic device 150 and may be visually output through a display of the haptic device 150.

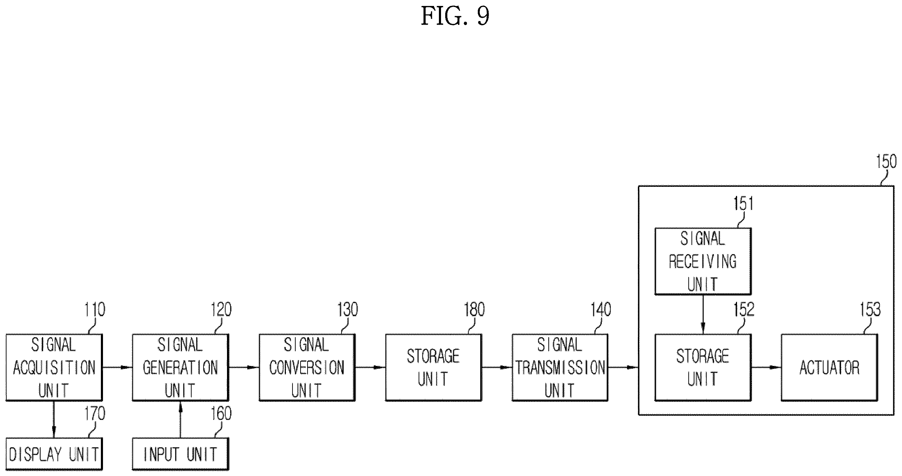

FIG. 9 is a control block diagram of the ultrasound system 100 to store and load a bio-signal or movement signal of a target object.

The ultrasound system 100 according to an embodiment of the present disclosure may store the bio-signal or the movement signal of the target object and may reload and output the bio-signal or movement signal as necessary. The ultrasound system 100 may further include a signal storage 180 to store a sensory signal converted by a signal converter 130, and the haptic device 150 may further include a signal storage 152 to store the sensory signal transmitted thereto.

The user may store the fetus ultrasound image and sensory signal acquired during diagnosis of a pregnant woman in the signal storage 180. When the user wants to recheck a state of the fetus or the pregnant woman wants to re-feel the fetal movement, the user may load and output the fetus ultrasound image and sensory signal.

In addition, when the haptic device 150 is implemented as a portable device such as a portable phone, the pregnant woman may receive the fetus ultrasound image and the sensory signal from the ultrasound diagnosis apparatus 101 and store the fetus ultrasound image and the sensory signal in the signal storage 152, and then load the fetus ultrasound image and the sensory signal and allow the sensory signal to be output through tactile sense such as vibration or the like through the haptic device 150 whenever she wants to feel the fetal movement.

As another method of haptically outputting movement of the target object, a movement oscillation wave is output along the movement of the target object. When this method is used, the haptic signal may be output such that vibration may flow along the movement of the target object.

In detail, when the target object is a fetus, if the fetus ultrasound image acquired by the signal acquisitioner 110 and the haptic signal generated by the signal converter 130 are transmitted to the haptic device 150, the fetus ultrasound image is displayed on the display 170 included in the haptic device 150 and the haptic signal is output as the movement oscillation wave in synchronization with the fetal movement of the fetus ultrasound image displayed on the display 170. That is, the vibration output through the display 170 may flow on a surface of the display 170 in synchronization with the fetal movement. In this case, the pregnant woman may more dynamically feel the fetal movement.

Response sensitivity may be set through the input 160 and refers to a degree by which the haptic device 150 responds to the bio-signal or movement signal of the target object. When the response sensitivity is set low, haptic sense is output low, and when the response sensitivity is set high, the haptic sense is output high.

In addition, it may be possible to determine a location of the target object where the bio-signal is generated, through the input 160. For example, when a fetal heartbeat signal is generated using the Doppler Effect, the location of the fetus heart may be automatically set by determining a location where frequency shift occurs, or alternatively, the user may directly set the location of the fetus heart through the input 160 by viewing the fetus ultrasound image.

In the above-described embodiments of the present disclosure, the signal converter 130 is included in the ultrasound diagnosis apparatus 101, which is merely an embodiment of the present. Alternatively, the signal converter 130 may be included in the haptic device 150. In this case, when the signal generator 120 generates the bio-signal or movement signal of the fetus and transmits the bio-signal or movement signal to the haptic device 150, the haptic device 150 may convert the bio-signal or movement signal into a sensory signal, such as, a haptic signal, a visual signal, an auditory signal, and the like.

Hereinafter, a method of controlling an ultrasound system will be described with regard to an embodiment of the present disclosure with reference to a flowchart.

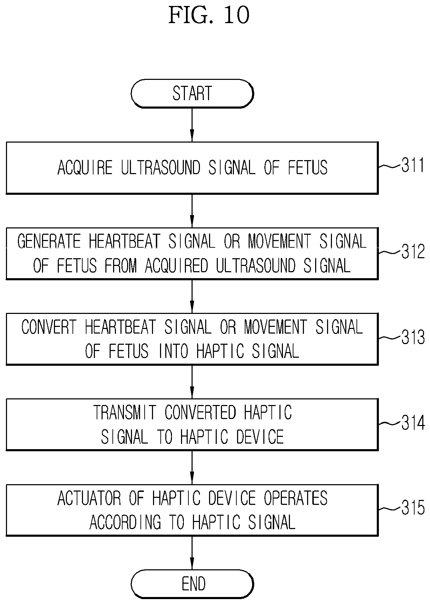

FIG. 10 is a flowchart of a method of controlling an ultrasound system, according to an embodiment of the present disclosure. For detailed description, in an embodiment of the present disclosure, a case in which a target object is a fetus will be described.

Referring to FIG. 10, an ultrasound signal of the fetus is acquired (311). The ultrasound signal may be acquired using a general method of acquiring an ultrasound signal by bringing an ultrasound probe into contact with the abdomen of a pregnant woman.

A fetal heartbeat signal or a movement signal is generated from the acquired ultrasound signal (312). The fetal heartbeat signal may be generated using the Doppler Effect. In this regard, the fetal heartbeat signal may be generated by extracting a signal, the frequency of which is shifted, from the ultrasound signal. The fetal movement signal will be described below.

In addition, the fetal heartbeat signal or the movement signal is converted into a sensory signal (313). When the heartbeat signal is converted, the heartbeat signal is converted into a simple signal such as a pulse signal. Then, the pulse signal may be converted into the sensory signal. The sensory signal may be a haptic signal such as vibration or may include the haptic signal and a visual signal or an auditory signal. In addition, the sensory signal may be stored, and then, may be loaded and transmitted to a haptic device as necessary.

When the converted haptic signal is transmitted to the haptic device (314), an actuator of the haptic device operates according to the sensory signal (315). For example, when the sensory signal is a haptic signal and the haptic device is a device that vibrates according to the haptic signal, the haptic device may vibrate according to the operation of the actuator, and a user may feel fetal heartbeat or fetal movement through the vibration.

As another example, the fetal heartbeat may be expressed by concentrating liquid on a specific location of the haptic device and repeating expansion and contraction according to the haptic signal.

If the sensory signal further includes a visual signal or an auditory signal, the visual signal may be output through a display included in the haptic device or the auditory signal may be output through a speaker. The visual signal may be a Doppler signal that is expressed in the form of fetus image signal or graph and the auditory signal may indicate heartbeat as a sound.

In this case, the user may set a response sensitivity. In addition, the haptic device may adjust an intensity of the sensory signal according to the set response sensitivity and may output the sensory signal. The sensory signal transmitted to the haptic device may be stored in the haptic device, and then, may be loaded to drive the actuator as necessary.

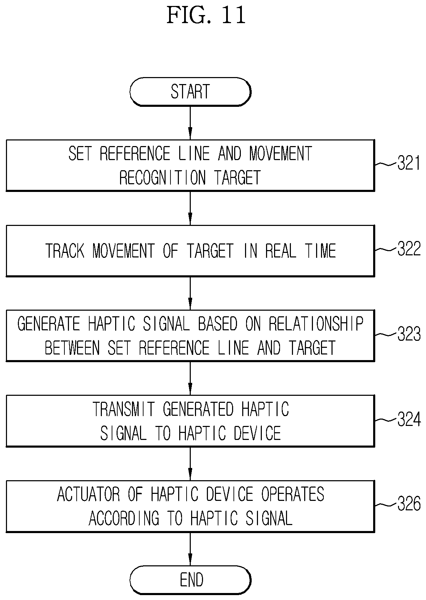

FIG. 11 is a flowchart of a method of controlling an ultrasound system to express fetal movement using a tracking algorithm and a collision detection algorithm. Referring to FIG. 11, a reference line for recognition of movement and a movement recognition target, movement of which is to be recognized are set (321). An ultrasound image of a target object may be displayed, and a user may set the desired reference line and movement recognition target on the displayed ultrasound image. In this case, a display for displaying the ultrasound image and an input used for user setting may be included in the ultrasound diagnosis apparatus or may be included in the haptic device.

When the setting is completed, movement of the set target object is tracked in real time (322). The movement of the movement recognition target may be tracked using a tracking algorithm such as particle tracking, Kalman tracking, Blob detection, or the like.

A haptic signal is generated based on a relationship between the reference line and the movement recognition target (323). As an example, when the relationship between the reference line and the movement recognition target satisfies a preset condition, a signal indicating that the movement recognition target moves, that is, a movement signal may be generated. The preset condition is a condition to determine whether the movement recognition target moves based on the reference line. For example, a case in which the reference line and the movement recognition target approach each other such that a distance there between may be equal to or less than a preset distance, or a case in which the movement recognition target reaches the reference line may be set as a condition for generation of the movement signal. In addition, the movement signal is converted into the haptic signal.

When the haptic signal is transmitted to the haptic device (324), the actuator of the haptic device operates according to the haptic signal (326). For example, when the haptic device is a device that vibrates according to the haptic signal, the haptic device may vibrate according to the operation of the actuator, and the user may feel fetal movement through the vibration.

The fetal movement signal may be converted into an auditory signal and may be transmitted to the haptic device 150 through the signal transmitter 140. Here, the auditory signal may be a signal indicating collision of the movement recognition target with the reference line or other sounds via which fetal movement may be detected. In addition, a fetus ultrasound image or a fetal movement image may also be transmitted to the haptic device 150 or may be visually output through a display of the haptic device 150.

As is apparent from the above description, a state of a target object may be felt through various senses such as visual, auditory, and haptic senses. In particular, when the target object is a fetus, a pregnant woman may feel fetal heartbeat or fetal movement through various senses such as visual, auditory, and haptic senses, thereby increasing the link between the fetus and the pregnant woman. In addition, fetal heartbeat or fetal movement may be implemented in a place such as a home rather than a hospital.

## Claims

1. An ultrasound system (100) comprising:
a signal acquisition unit (110) to acquire an ultrasound signal reflected from a fetus;
a signal generation unit (120) to generate a movement signal representing movement information of the fetus from the ultrasound signal by using a tracking algorithm;
a signal conversion unit (130) to convert the movement signal into a sensory signal for providing haptic output and at least one of visual output, and auditory output;
a signal transmission unit (140) to transmit the sensory signal to a haptic device (150),
a haptic device (150) to receive the sensory signal transmitted from the signal transmission unit (140) and to haptically and audibly outputs the sensory signal; and
an input unit (160) to set a reference line, and the fetus as a movement recognition target,
wherein, the signal generation unit (120) is configured to generate the movement signal based on whether a relationship between the reference line and the movement recognition target satisfies a preset condition.

2. The ultrasound system (100) according to claim 1, wherein the signal generation unit (120) generates the movement signal when the fetus and the reference line approach each other such that a distance there between is equal to or less than a preset distance.

3. The ultrasound system (100) according to claim 1, wherein the haptic device (150) visually outputs the ultrasound signal and the movement signal.

4. The ultrasound system (100) according to claim 3, further comprising an input unit (160) to set response sensitivity of the haptic device (150) with respect to the movement signal.

5. The ultrasound system (100) according to claim 1, further comprising a storage unit (180) to store the sensory signal, wherein the stored sensory signal is loaded from the storage unit (180) and transmitted to the haptic device (150) through the signal transmission unit (140).

6. The ultrasound system (100) according to claim 1, wherein the haptic device (150) comprises:
a signal receiving unit (151) to receive the sensory signal from the signal transmission unit (140);
a storage unit (152) to store the sensory signal; and
an actuator (153) driven according to the sensory signal when the sensory signal is loaded from the storage unit (152).

7. A method of controlling an ultrasound system (100), the method comprising:
acquiring an ultrasound signal reflected from a fetus;
generating a movement signal representing movement information of the fetus from the ultrasound signal by using a tracking algorithm;
converting the movement signal into a sensory signal for providing haptic output and at least one of visual output, and auditory output;
transmitting the sensory signal to a haptic device (150);
receiving the sensory signal transmitted from the signal transmission unit (140) and haptically and audibly outputting the sensory signal by the haptic device; and
wherein, the generating comprises setting a reference line and the fetus as a movement recognition target, and generating the movement signal based on a relationship between the reference line and the movement recognition target satisfies a preset condition.

## Patentansprüche

1. Ultraschallsystem (100), welches folgendes aufweist:
Eine Signalerlangungseinheit (110), um ein von einem Fötus reflektiertes Ultraschallsignal zu erlangen;
eine Signalerzeugungseinheit (120), um ein Bewegungssignal zu erzeugen, das Bewegungsinformationen des Fötus von dem Ultraschallsignal unter Verwendung eines Verfolgungsalgorithmus erzeugt;
eine Signalumwandlungseinheit (130), um das Bewegungssignal in ein Sensorsignal umzuwandeln, um eine haptische Ausgabe und wenigstens eines einer visuellen Ausgabe oder einer hörbaren Ausgabe zur Verfügung zu stellen;
eine Signalübertragungseinheit (140), um das Sensorsignal zu einer haptischen Vorrichtung (150) zu übertragen;
eine haptische Vorrichtung (150), um das von der Signalübertragungseinheit (140) übertragene Sensorsignal zu empfangen und das Sensorsignal haptisch und hörbar auszugeben; und
eine Eingabeeinheit (160), um eine Referenzlinie und den Fötus als ein Bewegungserkennungsziel festzulegen,
wobei die Signalerzeugungseinheit (120) dafür vorgesehen ist, das Bewegungssignal basierend darauf zu erzeugen, ob eine Beziehung zwischen der Referenzlinie und dem Bewegungserkennungsziel eine vorbestimmte Bedingung erfüllt.

2. Ultraschallsystem (100) nach Anspruch 1, wobei die Signalerzeugungseinheit (120) das Bewegungssignal erzeugt, wenn der Fötus und die Referenzlinie sich einander annähern, so dass ein Abstand zwischen denselben gleich oder geringer als ein voreingestellter Abstand ist.

3. Ultraschallsystem (100) nach Anspruch 1, wobei die haptische Vorrichtung (150) das Ultraschallsignal und das Bewegungssignal visuell ausgibt.

4. Ultraschallsystem (100) nach Anspruch 3, welches des Weiteren eine Eingabeeinheit (160) aufweist, um eine Antwort- bzw. Reaktionssensitivität der haptischen Vorrichtung (150) in Bezug auf das Bewegungssignal festzulegen.

5. Ultraschallsystem (100) nach Anspruch 1, welches des Weiteren eine Speichereinheit (180) zum Speichern des Sensorsignals aufweist, wobei das gespeicherte Sensorsignal von der Speichereinheit (180) geladen und durch die Signalübertragungseinheit (140) zu der haptischen Vorrichtung (150) übertragen wird.

6. Ultraschallsystem (100) nach Anspruch 1, wobei die haptische Vorrichtung (150) folgendes aufweist:
eine Signalempfangseinheit (151,) um das Sensorsignal von der Signalübertragungseinheit (140) zu empfangen;
eine Speichereinheit (152), um das Sensorsignal zu speichern; und
einen Aktuator (153), der gemäß dem Sensorsignal angetrieben wird, wenn das Sensorsignal von der Speichereinheit (152) geladen wird.

7. Verfahren zum Steuern eines Ultraschallsystems (100), wobei das Verfahren folgendes aufweist:
Erlangen eines von einem Fötus reflektierten Ultraschallsignals;
Erzeugen eines Bewegungssignals, welches Bewegungsinformationen des Fötus von dem Ultraschallsignal unter Verwendung eines Verfolgungsalgorithmus repräsentiert;
Umwandeln des Bewegungssignals in ein Sensorsignal, um eine haptische Ausgabe und wenigstens eines einer visuellen Ausgabe und einer hörbaren Ausgabe zur Verfügung zu stellen;
Übertragen des Sensorsignals zu einer haptischen Vorrichtung (150);
Empfangen des von der Signalübertragungseinheit (140) übertragenen Sensorsignals und haptisch und hörbar Ausgeben des Sensorsignals durch die haptische Vorrichtung; und
wobei das Erzeugen das Festlegen einer Referenzlinie und des Fötus als ein Bewegungserkennungsziel aufweist und das Erzeugen des Bewegungssignals basierend auf einer Beziehung zwischen der Referenzlinie und dem Bewegungserkennungssignal eine vorbestimmte Bedingung erfüllt.

## Revendications

1. Système ultrasonique (100) comportant :
une unité d'acquisition de signal ultrasonique (110) pour recevoir un signal ultrasonique réfléchi par un foetus;
une unité de génération de signal (120) pour générer un signal de mouvement représentant une information de mouvement du foetus à partir du signal ultrasonique en utilisant un algorithme de suivi ;
une unité de conversion de signal (130) pour convertir le signal de mouvement en un signal sensitif pour produire une sortie sensible au toucher et au moins une sortie visuelle et une sortie auditive;
une unité de transmission de signal (140) pour transmettre le signal sensitif a un dispositif sensible au toucher (150),
un dispositif sensible au toucher (150) pour recevoir le signal sensitif transmis par l'unité de transmission de signal (140) et les sorties sensible au toucher et auditive ; et
une unité d'entrée (160) pour fixer une ligne de référence, et le foetus comme étant une cible de reconnaissance de mouvement,
dans lequel l'unité de génération de signal (120) est configurée pour générer un signal de mouvement basé sur le fait qu'une relation entre la ligne de référence et la cible de reconnaissance de mouvement satisfait une condition prédéterminée.

2. Système ultrasonique (100) selon la revendication 1, dans lequel l'unité de génération de signal (120) génère un signal de mouvement lorsque le foetus et la ligne de référence se rapprochent l'un de l'autre de telle manière que la distance entre eux est égale ou inférieure à une distance prédéterminée.

3. Système ultrasonique (100) selon la revendication 1, dans lequel le dispositif sensible au toucher (150) émet visuellement le signal ultrasonique et le signal de mouvement.

4. Système ultrasonique (100) selon la revendication 3, comprenant en outre une unité d'entrée (160) pour fixer une réponse sensitive du dispositif sensible au toucher (150) par rapport au signal de mouvement.

5. Système ultrasonique (100) selon la revendication 1, comprenant en outre une unité de mémorisation (180) pour stocker le signal sensoriel ;
dans lequel le signal sensoriel stocké est chargé depuis l'unité de mémorisation (180) et
est transmis au dispositif sensible au toucher (150) par l'unité de transmission de signal (140).

6. Système ultrasonique (100) selon la revendication 1, dans lequel le dispositif sensible au toucher (150) comprend :
une unité de réception de signal (151) pour recevoir un signal sensoriel de l'unité de transmission de signal (140) ;
une unité de mémorisation (152) pour stocker le signal sensoriel ; et
un actuateur (153) piloté en fonction du signal sensitif lorsque le signal sensitif est chargé depuis l'unité de mémorisation (152).

7. Procédé de commande d'un système ultrasonique (100), le procédé comprenant :
l'acquisition d'un signal ultrasonique réfléchi par un foetus;
la génération d'un signal de mouvement représentant une information de mouvement du foetus à partir du signal ultrasonique en utilisant un algorithme de suivi de position ;
la conversion du signal de mouvement en un signal sensitif pour produire une sortie sensible au toucher et au moins une sortie visuelle et une sortie auditive;
la transmission du signal sensitif a un dispositif sensible au toucher (150),
la réception du signal sensitif transmis par l'unité de transmission de signal (140) et les sorties sensible au toucher et auditive ; et
dans lequel la génération comprend la fixation d'une ligne de référence et le foetus comme une cible de reconnaissance de mouvement et la génération du signal de mouvement basé sur une relation entre la ligne de référence et la reconnaissance de mouvement de la cible qui satisfait une condition prédéterminée.
